# FASCICULE DE BREVET EUROPEEN

(11) **EP 2 263 612 B1**
(45) Date de publication et mention de la délivrance du brevet: **28.09.2011**
(21) Numéro de dépôt: 10165828.4
(22) Date de dépôt: 14.06.2010
(51) Int. Cl.: A61F 2/36, A61F 2/30

(54) **Prothèse de hanche**
Hüftprothese
Hip prosthesis

(30) Priorité: 18.06.2009 FR 0954092
(43) Date de publication de la demande: 22.12.2010
(73) Titulaire: Cartillier, Jean-Claude, 69008 Lyon (FR); Jacquot, Laurent, 74370 Charvonnex (FR); Balay, Bruno, 01600 Trevoux (FR); Charlet, Claude, 69370 Saint Didier au Mont d'Or (FR); Fessy, Michel Henri, 69230 Saint Genis Laval (FR); Semay, Jean-Marc, 42270 Saint Priest en Jarez (FR); Setiey, Louis, 69400 Gleize (FR); Chatelet, Jean-Christophe, 01480 Jassans (FR); Vidalain, Jean-Pierre, 74940 Annecy le Vieux (FR); Machenaud, Alain, 74330 La Balme de Sillingy (FR); Ait Si Selmi, Tarik, 69006 Lyon (FR); Rollier, Jean-Charles, 74370 Saint Martin Bellevue (FR)
(72) Inventeur: Cartillier, Jean-Claude, 69008 Lyon (FR); Jacquot, Laurent, 74370 Charvonnex (FR); Balay, Bruno, 01600 Trevoux (FR); Charlet, Claude, 69370 Saint Didier au Mont d'Or (FR); Fessy, Michel Henri, 69230 Saint Genis Laval (FR); Semay, Jean-Marc, 42270 Saint Priest en Jarez (FR); Setiey, Louis, 69400 Gleize (FR); Chatelet, Jean-Christophe, 01480 Jassans (FR); Vidalain, Jean-Pierre, 74940 Annecy le Vieux (FR); Machenaud, Alain, 74330 La Balme de Sillingy (FR); Ait Si Selmi, Tarik, 69006 Lyon (FR); Rollier, Jean-Charles, 74370 Saint Martin Bellevue (FR)
(74) Mandataire: Maureau, Philippe

(56) Documents cités:
- FR-A- 2 111 202
- FR-A- 2 528 307
- FR-A- 2 832 310
- FR-A- 2 863 866
- FR-A- 2 867 381
- US-A- 5 163 964
- US-A1- 2004 013 703

## Description

La présente invention concerne une prothèse de hanche. Les caractéristiques du préambule de la revendication 1 sont connues du document FR-A-28 67 381.

Une prothèse de hanche comprend, de façon connue, une partie médullaire de forme allongée destinée à être engagée dans le canal médullaire d'un fémur, une partie formant col fémoral prothétique destinée à faire saillie du fémur et à être équipée d'une tête fémorale prothétique, et une collerette d'appui s'étendant transversalement à la partie formant col fémoral et destinée à venir prendre appui sur la corticale fémorale, et plus particulièrement sur l'éperon de Merckel, également dénommé calcar.

La présence de la collerette d'appui permet d'une part d'empêcher une pénétration de la partie médullaire dans le canal médullaire du patient au-delà d'une zone déterminée, et d'autre part d'améliorer l'assise de la prothèse.

Toutefois, une telle collerette d'appui a pour inconvénient de provoquer un pic de contraintes au niveau du calcar, ce qui peut générer une dégradation osseuse de ce dernier.

En outre, étant donné qu'une telle collerette d'appui recouvre la corticale interne du fémur et une partie des corticales antérieure et postérieure de ce dernier, elle empêche l'accès par le haut aux faces interne, antérieure et postérieure de la partie médullaire avec des ostéotomes. De ce fait, lorsque l'on souhaite extraire la prothèse de hanche, il est nécessaire de réaliser une résection du fémur en dessous de la collerette d'appui afin de pouvoir atteindre les faces interne, antérieure et postérieure de la partie médullaire avec des ostéotomes destinés à désolidariser la partie médullaire du fémur. Il en résulte une dégradation osseuse du fémur et une intervention chirurgicale complexe.

La présente invention vise à remédier à ces inconvénients.

Le problème technique à la base de l'invention consiste donc à fournir une prothèse de hanche qui soit de structure simple et économique, qui permette de simplifier et d'améliorer l'intervention chirurgicale, tout en limitant les contraintes appliquées sur le calcar.

A cet effet, l'invention concerne une prothèse de hanche comprenant une partie médullaire de forme allongée destinée à être engagée dans le canal médullaire d'un fémur, une partie formant col fémoral prothétique destinée à faire saillie du fémur et à être équipée d'une tête fémorale prothétique, et une collerette d'appui s'étendant transversalement à la partie formant col fémoral et destinée à venir prendre appui sur la corticale fémorale, la collerette d'appui comprenant au moins une portion médiale faisant saillie de la face interne de la partie médullaire et des portions latérales faisant saillie respectivement des faces antérieure et postérieure de la partie médullaire, caractérisée en ce que la collerette d'appui comporte une lumière s'étendant sur la portion médiale de la collerette d'appui et en outre sur les portions latérales de la collerette d'appui.

La présence d'une lumière sur la collerette d'appui permet de visualiser l'appui effectif de cette dernière sur la corticale fémorale, et donc d'assurer un parfait positionnement de la partie médullaire.

La présence d'une telle lumière sur la collerette d'appui permet en outre de contrôler visuellement l'éventuelle présence de fissure ou de fracture du calcar après mise en place de la partie médullaire, et donc de prendre les dispositions nécessaires le plus rapidement possible (par exemple réaliser un cerclage du fémur) afin de permettre une consolidation rapide de cette fissure ou fracture.

De plus, la présence d'une telle lumière sur la collerette d'appui rend cette dernière plus souple, ce qui permet de modifier les contraintes appliquées par la collerette d'appui sur le calcar, et notamment d'éviter les pics de contraintes sur ce dernier.

Le fait que la lumière s'étende sur la portion médiale de la collerette d'appui et en outre sur les portions latérales de cette dernière permet un accès par le haut aux faces interne, antérieure et postérieure de la partie médullaire avec des ostéotomes. Il en résulte une intervention chirurgicale simplifiée en cas de reprise de la prothèse de hanche.

Avantageusement, la lumière est agencée pour permettre le passage d'ostéotomes, tels que des lames d'ostéotomie par exemple plates, incurvées ou en forme de gouge.

De préférence, la face inférieure de la collerette d'appui est revêtue d'un revêtement de phosphate de calcium, tel que l'hydroxyapatite et le phosphate tricalcique.

De façon préférentielle, la lumière s'étend sensiblement perpendiculairement à la face inférieure de la collerette d'appui.

Préférentiellement, la lumière présente sensiblement une forme de haricot.

Selon une caractéristique de l'invention, l'un des bords de la lumière s'étend dans la continuité de la face interne de la partie médullaire. Ces dispositions permettent d'assurer un guidage des ostéotomes le long de la lumière et d'éviter à ces derniers de venir en butée contre la face interne de la partie médullaire et contre le rebord du calcar.

Avantageusement, la prothèse comprend un obturateur amovible agencé pour obturer la lumière. Un tel obturateur est destiné à d'obturer la lumière après insertion de la partie médullaire dans le canal médullaire du fémur et vérification que cette dernière est parfaitement positionnée et qu'il n'y a aucune fissure du calcar. Ces dispositions permettent d'éviter la migration de particules d'usure de la prothèse en direction de l'interface partie médullaire-fémur, et donc la création d'ostéolyses susceptibles de provoquer le descellement de la prothèse. Ces dispositions permettent également de former un obstacle au saignement fémoral.

Selon une autre caractéristique de l'invention, la collerette d'appui est amovible. De préférence, la collerette d'appui présente la forme d'un étrier en U comportant deux branches de blocage solidaires de chaque extrémité d'une partie centrale, les branches de blocage étant destinées à être engagées respectivement dans des rainures complémentaires ménagées dans les faces antérieure et postérieure de la partie médullaire, et la partie centrale étant destinée à venir en butée contre la face interne de la partie médullaire.

De façon avantageuse, la collerette d'appui est située au niveau de la jonction entre la partie médullaire et la partie formant col fémoral.

Selon un mode de réalisation de l'invention, la prothèse de hanche comporte au moins une cale destinée à être positionnée sous la collerette d'appui de manière à être interposée entre cette dernière et le fémur, la cale comportant des moyens de fixation agencés pour coopérer avec la lumière ménagée sur la collerette d'appui.

Selon un mode de réalisation de l'invention, la prothèse de hanche comporte un dispositif de diffusion d'au moins une substance médicamenteuse, telle qu'un antibiotique, destiné à être monté dans la lumière ménagée sur la collerette d'appui. Le fait de positionner un tel dispositif de diffusion dans la lumière ménagée dans la collerette d'appui permet de diffuser localement une substance médicamenteuse, ce qui évite d'injecter par voie intra-veineuse une dose importante de substance médicamenteuse au patient. De plus, ce positionnement du dispositif de diffusion dans la lumière assure la diffusion d'une substance médicamenteuse d'une part en direction du canal médullaire et d'autre part en direction de la cavité articulaire.

Le dispositif de diffusion comporte de façon avantageuse une portion présentant une forme au moins partiellement complémentaire de la lumière. Le dispositif de diffusion peut être par exemple un comprimé solide ou une capsule, de préférence résorbable, enfermant au moins une substance médicamenteuse.

De toute façon l'invention sera bien comprise à l'aide de la description qui suit en référence au dessin schématique annexé représentant, à titre d'exemple non limitatif, une forme d'exécution de cette prothèse de hanche.
Figure 1 est une vue partielle en perspective d'une prothèse de hanche selon l'invention.
Figure 2 est une vue partielle de profil de la prothèse de figure 1.
Figure 3 est une vue en perspective d'une cale selon un premier mode de réalisation de l'invention.
Figure 4 est une vue en perspective d'une cale selon un second mode de réalisation de l'invention.
Figure 5 est une vue en perspective d'un dispositif de diffusion selon l'invention.

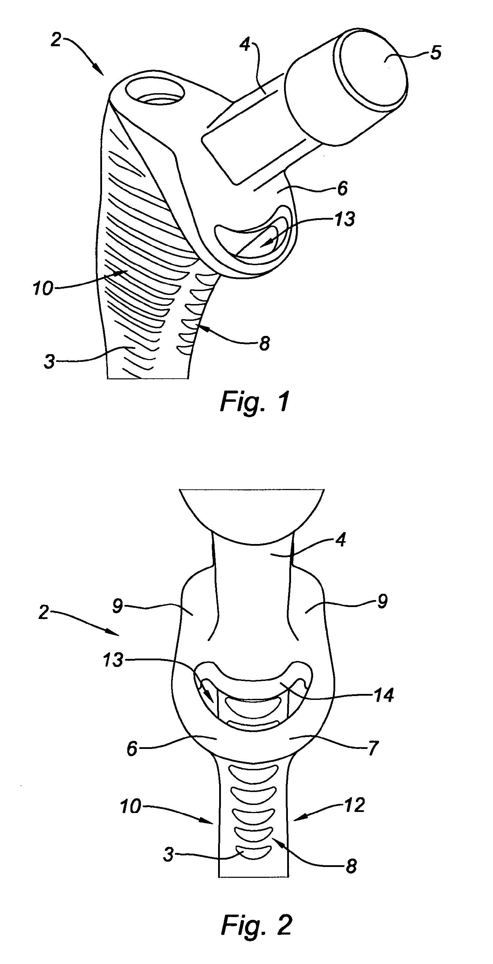

Les figures 1 et 2 représentent une prothèse de hanche 2 réalisée de préférence en titane, en alliage de titane ou en chrome cobalt.

La prothèse de hanche 2 comprend une partie médullaire 3 de forme allongée destinée à être engagée dans le canal médullaire d'un fémur, et une partie formant col fémoral prothétique 4 destinée à faire saillie du fémur et à être équipée d'une tête fémorale prothétique (non représentée sur les figures).

La partie formant col fémoral 4 comporte une extrémité proximale tronconique 5 agencée pour coopérer avec une cavité tronconique de forme complémentaire ménagée dans la tête fémorale prothétique.

La prothèse de hanche 2 comprend en outre une collerette d'appui 6 située au niveau de la jonction entre la partie médullaire 3 et la partie formant col fémoral 4.

La collerette d'appui 6 s'étend sensiblement perpendiculairement à la partie formant col fémoral 4. La collerette d'appui 6 s'étend également sensiblement selon un angle d'environ 45° par rapport à l'axe diaphysaire.

La collerette d'appui 6 est destinée à venir prendre appui sur la corticale interne du fémur après section de celui-ci et enfoncement de la partie médullaire 3 dans le canal médullaire. La collerette d'appui 6 est plus particulièrement destinée à venir prendre appui sur l'éperon de Merckel, également dénommé calcar.

Comme montré plus particulièrement sur la figure 2, la collerette d'appui 6 comprend une portion médiale 7 faisant saillie de la face interne 8 de la partie médullaire 3 et des portions latérales 9 faisant saillie respectivement des faces antérieure 10 et postérieure 12 de la partie médullaire 3. Il convient de noter que la face interne 8 de la partie médullaire 3 est destinée à être tournée vers la calcar, tandis que sa face externe est destinée à être tournée vers le grand trochanter, les faces antérieure et postérieure reliant les faces interne et externe de la partie médullaire 3.

La collerette d'appui 6 comporte une lumière 13 présentant une forme de haricot. La lumière 13 s'étend sur la portion médiale 7 et les portions latérale 9 de la collerette d'appui 6. La longueur de la lumière 13 selon un plan antéro-postérieur est de préférence comprise entre 3 et 5 mm.

La lumière 13 s'étend sensiblement perpendiculairement à la face inférieure de la collerette d'appui 6. Le bord 14 de la lumière 6 situé à proximité de la partie formant col fémoral 4 s'étend dans la continuité de la face interne 8 de la partie médullaire 3.

La lumière 13 est agencée pour permettre le passage d'ostéotomes, tels que des lames d'ostéotomie souples.

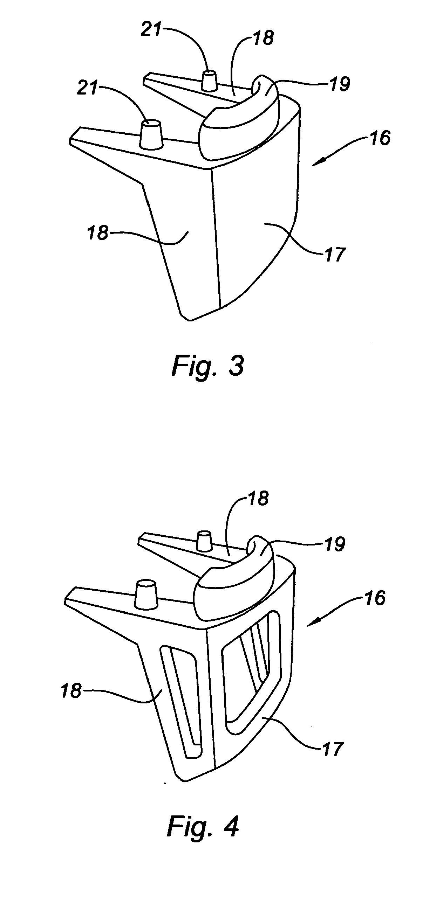

Comme montré sur la figure 3, la prothèse de hanche comprend également une cale 16 destinée à être positionnée sous la collerette d'appui 6 de manière à être interposée entre cette dernière et le fémur. La cale 16 permet de compenser des pertes osseuses, notamment du calcar, et ainsi de reconstituer des appuis pour la prothèse de hanche. La cale 16 est de préférence réalisée en titane.

La cale 16 présente la forme d'un étrier en U comportant une portion centrale 17 destinée à s'étendre sous la portion médiale 7 de la collerette d'appui 6, et deux branches latérales 18 solidaires de la portion centrale 17 et destinées à s'étendre respectivement sous les portions latérales 9 de la collerette d'appui 6.

La portion centrale 17 présente un profil complémentaire de celui de la face interne 8 de la partie médullaire 3 et est destinée à venir en butée contre la face interne 8 de cette dernière.

Afin d'assurer une fixation de la cale 16 sur la collerette d'appui 6, la cale 16 comprend d'une part un organe 19 faisant saillie de la face supérieure de la portion centrale 17 de la cale et agencé pour s'engager dans la lumière 13, et d'autre part deux ergots 21 faisant saillie respectivement de la face supérieure de chaque branche latérale 18 de la cale et agencés pour coopérer avec deux orifices complémentaires (non représentés sur les figures) ménagés respectivement sur la face inférieure des portions latérales 9 de la collerette d'appui 6.

Selon une variante de réalisation montrée sur la figure 4, les branches latérales 18 et la portion centrale 17 de la cale16 sont ajourées afin de favoriser la repousse osseuse et également de permettre la fixation de greffe osseuse ou de substitut osseux.

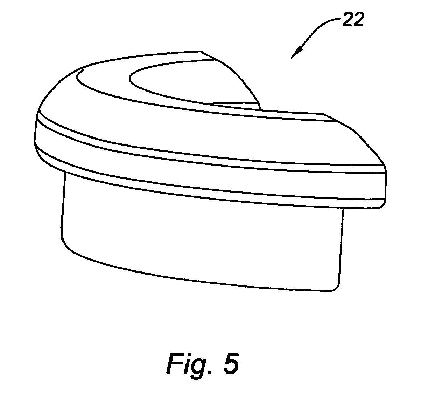

Comme montré sur la figure 5, la prothèse de hanche comprend également un dispositif de diffusion 22 d'au moins une substance médicamenteuse, telle qu'un antibiotique, destiné à être monté dans la lumière 13 ménagée sur la collerette d'appui. Le dispositif de diffusion 22 comporte une portion 23 présentant une forme au moins partiellement complémentaire de la lumière 13.

Le dispositif de diffusion peut être par exemple un comprimé solide ou une capsule, de préférence résorbable, enfermant une ou plusieurs substances médicamenteuses.

Selon une variante de réalisation de l'invention, la collerette d'appui 6 est amovible. Selon cette variante de réalisation, la collerette d'appui présente la forme d'un étrier en U comportant deux branches de blocage solidaires de chaque extrémité d'une partie centrale. Les branches de blocage de la collerette d'appui sont destinées à être engagées respectivement dans des rainures complémentaires ménagées dans les faces antérieure et postérieure de la partie médullaire, chaque rainure étant inclinée de bas en haut et de la face interne vers la face externe de la partie médullaire. La partie centrale de la collerette d'appui est destinée à venir en butée contre la face interne de la partie médullaire.

Selon un mode de réalisation de l'invention non représenté sur les figures, la prothèse de hanche comprend un obturateur amovible agencé pour obturer la lumière ménagée sur la collerette d'appui.

Selon un autre mode de réalisation de l'invention non représenté sur les figures, la partie médullaire et la partie formant col fémoral pourraient ne pas être monobloc. Ainsi, la partie formant col fémoral pourrait être amovible et comporter une partie centrale sensiblement cylindrique, une extrémité distale tronconique agencée pour coopérer avec une cavité tronconique de forme complémentaire ménagée dans l'extrémité proximale de la partie médullaire, et une extrémité proximale tronconique agencée pour coopérer avec une cavité tronconique de forme complémentaire ménagée dans la tête fémorale prothétique.

Comme il va de soi, l'invention ne se limite pas à la seule forme d'exécution de cette prothèse de hanche, décrite ci-dessus à titre d'exemple, elle en embrasse au contraire toutes les variantes de réalisation. C'est ainsi notamment que la prothèse de hanche selon l'invention pourrait être une prothèse de hanche à fixation biologique ou à fixation cimentée.

## Revendications

1. Prothèse de hanche (2) comprenant une partie médullaire (3) de forme allongée destinée à être engagée dans le canal médullaire d'un fémur, une partie formant col fémoral prothétique (4) destinée à faire saillie du fémur et à être équipée d'une tête fémorale prothétique, et une collerette d'appui (6) s'étendant transversalement à la partie formant col fémoral et destinée à venir prendre appui sur la corticale fémorale, la collerette d'appui (6) comprenant au moins une portion médiale (7) faisant saillie de la face interne (8) de la partie médullaire (3) et des portions latérales (9) faisant saillie respectivement des faces antérieure et postérieure de la partie médullaire (3), **caractérisée en ce que** la collerette d'appui (6) comporte une lumière (13) s'étendant sur la portion médiale de la collerette d'appui et en outre sur les portions latérales de la collerette d'appui.

2. Prothèse selon la revendication 1, **caractérisée en ce que** la lumière (13) s'étend sensiblement perpendiculairement à la face inférieure de la collerette d'appui (6).

3. Prothèse selon la revendication 1 ou 2, **caractérisée en ce que** la lumière (13) présente sensiblement une forme de haricot.

4. Prothèse selon l'une des revendications 1 à 3, **caractérisée en ce que** l'un (14) des bords de la lumière (13) s'étend dans la continuité de la face interne (8) de la partie médullaire (3).

5. Prothèse selon l'une des revendications 1 à 4, **caractérisée en ce que** la collerette d'appui (6) est amovible.

6. Prothèse selon la revendication 5, **caractérisée en ce que** la collerette d'appui présente la forme d'un étrier en U comportant deux branches de blocage solidaires de chaque extrémité d'une partie centrale, les branches de blocage étant destinées à être engagées respectivement dans des rainures complémentaires ménagées dans les faces antérieure et postérieure de la partie médullaire, et la partie centrale étant destinée à venir en butée contre la face interne de la partie médullaire.

7. Prothèse selon l'une des revendications 1 à 6, **caractérisée en ce qu'**elle comprend un obturateur amovible agencé pour obturer la lumière.

8. Prothèse selon l'une des revendications 1 à 7, **caractérisée en ce qu'**elle comporte au moins une cale (16) destinée à être positionnée sous la collerette d'appui (6) de manière à être interposée entre cette dernière et le fémur, la cale (16) comportant des moyens de fixation (19) agencés pour coopérer avec la lumière (13) ménagée sur la collerette d'appui.

9. Prothèse selon l'une des revendications 1 à 8, **caractérisée en ce qu'**elle comporte un dispositif de diffusion d'au moins une substance médicamenteuse, telle qu'un antibiotique, destiné à être monté dans la lumière (13) ménagée sur la collerette d'appui.

10. Prothèse selon la revendication 9, **caractérisée en ce que** le dispositif de diffusion comporte une portion présentant une forme au moins partiellement complémentaire de la lumière (13).

11. Prothèse selon la revendication 9 ou 10, **caractérisée en ce que** le dispositif de diffusion est un comprimé solide ou une capsule enfermant au moins une substance médicamenteuse.

## Claims

1. A hip prosthesis (2) comprising a medullar portion (3) of an elongated shape intended to be engaged into the medullar channel of a femur, a portion forming a prosthetic femoral neck (4) intended to protrude from the femur and to be equipped with a prosthetic femoral head, and a supporting flange (6), extending transversely to the portion forming a femoral head and intended to bear upon the femoral cortical bone, the supporting flange (6) comprising at least one medial portion (7) protruding from the internal face (8) of the medullar portion (3) and lateral portions (9) protruding from the anterior and posterior faces of the medullar portion (3), respectively, **characterized in that** the supporting flange (6) includes a lumen (13), extending over the medial portion of the supporting flange and further over the lateral portions of the supporting flange.

2. The prosthesis according to claim 1, **characterized in that** the lumen (13) substantially extends perpendicularly to the lower face of the supporting flange (6).

3. The prosthesis according to claim 1 or 2, **characterized in that** the lumen (13) substantially has the shape of a bean.

4. The prosthesis according to one of claims 1 to 3, **characterized in that** one (14) of the edges of the lumen (13) extends in the continuity of the internal face (8) of the medullar portion (3).

5. The prosthesis according to one of claims 1 to 4, **characterized in that** the supporting flange (6) is removable.

6. The prosthesis according to claim 5, **characterized in that** the supporting flange has the shape of a U-yoke including two blocking branches secured to each end of a central portion, the blocking branches being respectively intended to be engaged in mating grooves made in the anterior and posterior faces of the medullar portion, and the central portion being intended to abut against the internal face of the medullar portion.

7. The prosthesis according to one of claims 1 to 6, **characterized in that** it comprises a removable obturator laid out in order to obturate the lumen.

8. The prosthesis according to one of claims 1 to 7, **characterized in that** it includes at least one wedge (16) intended to be positioned under the supporting flange (6) so as to be interposed between the latter and the femur, the wedge (16) including an attachment means (19) laid out so as to cooperate with the lumen (13) made on the supporting flange.

9. The prosthesis according to one of claims 1 to 8, **characterized in that** it includes a device for diffusing at least one medicinal substance, such as an antibiotic, intended to be mounted in the lumen (13) made on the supporting flange.

10. The prosthesis according to claim 9, **characterized in that** the diffusion device includes a portion having a shape which at least partly mates the lumen (13).

11. The prosthesis according to claim 9 or 10, **characterized in that** the diffusion device is a solid tablet or a capsule containing at least one medicinal substance.

## Patentansprüche

1. Hüftprothese (2), die einen länglich geformten medullären Abschnitt (3) umfasst, der dazu bestimmt ist, in den medullären Kanal eines Oberschenkels eingepasst zu sein, einen den Prothesenoberschenkelhals bildenden Abschnitt (4), der dazu bestimmt ist, aus dem Oberschenkel hervorzustehen und mit einem Prothesen-Oberschenkelkopf ausgestattet zu sein, und einen Abstützring (6), der sich transversal zu dem Abschnitt erstreckt, der den Oberschenkelhals bildet und dazu bestimmt, sich auf der Oberschenkelrinde abzustützen, wobei der Abstützring (6) mindestens einen mittleren Abschnitt (7) umfasst, der von der Innenseite (8) des medullären Abschnitts (3) hervorsteht und seitliche Abschnitte (9), die von den jeweils vorderen und hinteren Seiten des medullären Abschnitts (3) hervorstehen, **dadurch gekennzeichnet**, das der Abstützring (6) eine Öffnung (13) aufweist, die sich über den mittleren Abschnitt des Abstützrings erstreckt und weiterhin über die seitlichen Abschnitte des Abstützrings.

2. Prothese nach Anspruch 1, **dadurch gekennzeichnet, dass** sich die Öffnung (13) etwa senkrecht zur Unterseite des Abstützrings (6) erstreckt.

3. Prothese nach Anspruch 1 oder 2, **dadurch gekennzeichnet, dass** die Öffnung (13) etwa bohnenförmig ist.

4. Prothese nach einem der Ansprüche 1 bis 3, **dadurch gekennzeichnet, dass** sich einer (14) der Ränder der Öffnung (13) in Fortsetzung der Innenseite (8) des medullären Abschnitts (3) erstreckt.

5. Prothese nach einem der Ansprüche 1 bis 4, **dadurch gekennzeichnet, dass** der Abstützring (6) abnehmbar ist.

6. Prothese nach Anspruch 5, **dadurch gekennzeichnet, dass** der Abstützring die Form eines U-förmigen Bügels aufweist, der zwei Blockierarme aufweist, die mit jedem Ende eines zentralen Abschnitts verbunden sind, wobei die Blockierarme dazu bestimmt sind, jeweils in komplementäre Rillen einzugreifen, die in die vordere und hintere Seite des medullären Abschnitts eingearbeitet sind, und der zentrale Abschnitt dazu bestimmt ist, an die Innenseite des medullären Abschnitts anzuschlagen.

7. Prothese nach einem der Ansprüche 1 bis 6, **dadurch gekennzeichnet, dass** sie einen abnehmbaren Verschluss umfasst, der ausgebildet ist, um die Öffnung zu verschließen.

8. Prothese nach einem der Ansprüche 1 bis 7, **dadurch gekennzeichnet, dass** sie mindestens einen Keil (16) aufweist, der dazu bestimmt ist, unter dem Abstützring (6) derart platziert zu sein, dass er sich zwischen diesem und dem Oberschenkel befindet, wobei der Keil (16) Befestigungsmittel (19) aufweist, die ausgebildet sind, um mit der in den Abstützring eingearbeiteten Öffnung (13) zusammenzuarbeiten.

9. Prothese nach einem der Ansprüche 1 bis 8, **dadurch gekennzeichnet, dass** sie eine Verteilervorrichtung mindestens einer medikamentösen Substanz aufweist, wie zum Beispiel eines Antibiotikums, die dazu bestimmt ist, in die in den Abstützring eingearbeitete Öffnung (13) eingesetzt zu sein.

10. Prothese nach Anspruch 9, **dadurch gekennzeichnet, dass** die Verteilervorrichtung einen Abschnitt umfasst, der eine Form aufweist, die zumindest teilweise zu der Öffnung (13) komplementär ist.

11. Prothese nach Anspruch 9 oder 10, **dadurch gekennzeichnet, dass** die Verteilervorrichtung eine feste Tablette oder eine Kapsel ist, die mindestens eine medikamentöse Substanz einschließt.
